Europäisches Patentamt

European Patent Office

Office européen des brevets

(19)

(11) Publication number: **0 247 723**
**A2**

(12) # EUROPEAN PATENT APPLICATION

(21) Application number: **87303498.7**

(22) Date of filing: **22.04.87**

(51) Int. Cl.⁴: **A 61 K 31/19,** A 61 K 31/54, A 61 K 31/34

(30) Priority: **28.04.86 CA 507712**

(43) Date of publication of application: **02.12.87**
**Bulletin 87/49**

(84) Designated Contracting States: **CH DE FR GB IT LI NL**

(71) Applicant: **MERCK FROSST CANADA INC.,**
**16711 Trans-Canada Highway, Kirkland Quebec (CA)**

(72) Inventor: **Ford-Hutchinson, Anthony, 69 Hyde Park,**
**Beaconfield Quebec H9W 5L7 (CA)**

(74) Representative: **Hesketh, Alan, Dr., European Patent**
**Department Merck & Co., Inc. Terlings Park Eastwick**
**Road, Harlow Essex, CM20 2QR (GB)**

(54) **Compositions and methods for treating allergic conjuctivitis.**

(57) Therapeutic compositions are disclosed for treating allergic conjunctivitis. The compositions comprise certain $LTD_4$ antagonists and synthesis inhibitors.

EP 0 247 723 A2

ACTORUM AG

## TITLE OF THE INVENTION
COMPOSITIONS AND METHODS FOR TREATING ALLERGIC CONJUNCTIVITIS

## BACKGROUND OF THE INVENTION

Allergic conjunctivitis is an acute inflammation of the conjunctiva. It is usually treated with topical corticosteroids, or oral antihistamines, optionally with a topical vasoconstrictor.

## SUMMARY OF THE INVENTION

It has now been found that several $LTD_4$ antagonists and leukotriene biosynthesis inhibitors are particularly useful in the treatment of allergic conjunctivitis.

## DETAILED DESCRIPTION

The leukotriene antagonists and biosynthesis inhibitors useful in the compositions and methods of this invention are:

3369P/1126A      - 2 -      17404

I   4-((3-(4-acetyl-3-hydroxy-2-propylphenoxy)-propyl)sulfonyl)-Y-oxobenzene butanoic acid;

II   (ßS*,YR*)-4-(3-(4-acetyl-3-hydroxy-2-propyl-phenoxy)propylthio)-Y-hydroxy-ß-methylbenzene-butanoic acid;

III   4-bromo-2,7-dimethoxy-3H-phenothiazin-3-one; and

IV   2,3-dihydro-6-(3-(2-hydroxymethyl)phenyl-2-propenyl)-5-benzofuranol;

and the ophthalmologically acceptable salts thereof.

These compounds are described in the following EP patent applications:

| Compound | Reference | Pub. Date |
|----------|-----------|-----------|
| I | EP 104,885 | April 4, 1984 |
| II | EP 104,885 | April 4, 1984 |
| III | EP 115,394 | August 8, 1984 |
| IV | EP 143,952 | June 12, 1985 |

The most preferred species is 2,3-dihydro-6-(3-(2-hydroxymethyl)phenyl-2-propenyl)-5-benzofuranol.

The pharmaceutical preparation which contains the active compound may be conveniently admixed with a non-toxic pharmaceutical organic carrier, or with a non-toxic pharmaceutical inorganic carrier. Typical of pharmaceutically acceptable carriers are, for example, water, mixtures of water and water-miscible solvents such as lower alkanols or aralkanols, vegetable oils, polyalkylene glycols, petroleum based jelly, ethyl cellulose, ethyl oleate, carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenylethanol, buffering ingredients such as sodium chloride, sodium borate, sodium acetate, gluconate buffers, and other conventional ingredients such as sorbitan monolaurate, triethanolamine oleate, polyoxyethylene sorbitan monopalmitate, dioctyl sodium sulfosuccinate, monothioglycerol, thiosorbitol, ethylenediamine tetracetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles, aqueous gel vehicles and the like. Generally, the drug is present in such vehicles in an amount of from 0.0001 to about 10% by weight or more. Preferably the drug is present in an amount of from 0.001 to 3% by weight, and most preferably from 0.03 to 1% by weight.

While many patients find liquid medication to be entirely satisfactory, others may prefer a solid medicament that is topically applied to the eye, for example, a solid dosage form that is suitable for insertion into the cul-de-sac. To this end the active compound can be included with a non-bioerodible insert, i.e. one which after dispensing the drug remains essentially intact, or a bioerodible insert, i.e. one that either is soluble in lacrimal fluids, or otherwise disintegrates.

While the insert employed is not critical, those disclosed in 3,630,200 Higuchi; 3,811,444 Heller et al.; 4,177,256 Michaels et al.; 3,868,445 Ryde et al.; 3,845,201 Haddad; 3,981,303 Higuchi; and 3,867,519 Michaels, are satisfactory; in general, however, the insert described below is found preferable.

For example, one may use a solid water soluble polymer as the carrier for the medicament. The polymer used to form the insert may be any water soluble non-toxic polymer, for example, cellulose derivatives such as methylcellulose, sodium carboxymethyl cellulose, or a hydroxy lower alkyl cellulose such as hydroxyethyl cellulose, hydroxypropyl cellulose, hydroxypropylmethyl cellulose and the like; acrylates such as polyacrylic acid salts, ethyl acrylates, polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum, and mixtures of said polymer.

It is highly preferred that the solid inserts of this invention are available for use by the patient in a pathogen free condition. Thus, it is preferred to sterilize the inserts and so as insure against recontamination, the sterilization is preferably conducted after packaging. The best mode of sterilizing is to employ ionizing irradiation including irradiation emanating from Cobalt 60 or high energy electron beams.

Drops are used in the usual way employing one to two drops per eye per patient per day. When inserts are employed usually one insert per patient per eye per day is satisfactory. An anti-allergic dose can be as little as 1-100 ng to as much as 0.1-1 mg per eye per patient per day of active medicament. As the individual differences between patient drug response are encountered and as experience with the medicament increases and information accumulates because of a larger patient population being developed, the daily ocular dose for the median population group can be stated with greater statistical accuracy. It may well be found that only a few patients respond to the minimal dose, and then only for a transient period. Also only a few patients may require administration of the drug at the higher dosage ranges. The dose also may be divided for administration. Thus, the quantities set forth previously can be administered in a course of individual deliveries comprising 1-4 or more times per day.

The concentration of active drug in any formulation can vary within a wide range. Clearly, as a function of concentration, the desired dose of formulation will consequently vary for example from a single drop or insert or multiple drops or inserts or larger or smaller inserts.

The following examples of ophthalmic formulations are given by way of illustration.

<p style="text-align:center">EXAMPLE 1</p>

### Solution Composition

| | a | b |
|---|---|---|
| Compound I, II, III, or IV | 1 mg. | 15 mg. |
| Monobasic sodium phosphate .2$H_2O$ | 9.38 mg. | 6.10 mg. |
| Dibasic sodium phosphate .12$H_2O$ | 28.48 mg. | 16.80 mg. |
| Benzalkonium chloride | 0.10 mg. | 0.10 mg. |
| Water for injection q.s. ad. | 1.0 ml. | 1.0 ml. |

Compound I, II, III, or IV, phosphate buffer salts, and benzalkonium chloride are added to and admixed with water. The pH of the resulting admixture is adjusted to 6.8 and the final formulation diluted to volume. The formulation is rendered sterile by appropriate means, such as starting the preparative procedure with sterile components and proceeding under sterile conditions, irradiating or autoclaving the finished formulation, or the like.

## EXAMPLE 2

Compound I, II, III, or IV                    5 mg.

petrolatum _q.s._ _ad._                       1 gram

Compound I, II, III, or IV and the
petrolatum are aseptically combined.

## EXAMPLE 3

Compound I, II, III, or IV                    1 mg.

Hydroxypropyl cellulose or
    hydroxypropylmethyl cellulose _q.s._     12 mg.

Ophthalmic inserts are manufactured from
compression molded films which are prepared on a
Carver Press by subjecting the powdered mixture of
the above ingredients to a compressional force of
12,000 lbs. (gauge) at 300°F (149°C) for one to four
minutes. The film is cooled under pressure by having
cold water circulate in the platen. Ophthalmic
inserts are then individually cut from the film with
a rod-shaped punch. Each insert is placed into a
vial, which is then placed in a humidity cabinet (88%
R.H. at 30°C) for two to four days. After removal
from the humidity cabinet, the vials are stoppered
and then capped. The vials containing the hydrate
insert are then autoclaved at 250°F (121°C) for 1/2
hour.

## EXAMPLE 4

Compound I, II, III, or IV                    1 mg.

Hydroxypropyl cellulose q.s. ad.                    12 mg.

Ophthalmic inserts are manufactured from a solvent cast film prepared by making a viscous solution of the powdered ingredients listed above using methanol as the solvent. The solution is placed on a Teflon plate and allowed to dry at ambient conditions. After drying, the film is placed in an 88% R. H. cabinet until it is pliable. Appropriately sized inserts are cut from the film.

## EXAMPLE 5

Compound I, II, III, or IV                    1 mg.

Hydroxypropylmethyl cellulose
    q.s. ad.                    12 mg.

Ophthalmic inserts are manufactured from a solvent cast film which is prepared by making a viscous solution of the powdered blend of the above ingredients using a methanol/water solvent system (10 ml. methanol is added to 2.5 g. of the powdered blend, to which 11 ml. of water (in three divided portions) is added. The solution is placed on a Teflon plate and allowed to dry at ambient conditions. After drying, the film is placed in an 88% R. H. cabinet until it is pliable. Appropriately sized inserts are then cut from the film.

### EXAMPLE 6
### AQUEOUS GEL

Compound I, II, III, or IV                                    1 mg.

Water                                                          99 mg.

Carbopol 934 (B.F. Goodrich, Cleveland)                       1 mg.

### EXAMPLE 7
### INHIBITION OF ANTIGEN-INDUCED OCULAR INFLAMMATION

Guinea-pigs were actively sensitized to produce $IgG_1$ and IgE antibodies to ovalbumin (2 weeks). Animals were then challenged topically in the eyes (10 µl of 2.5% w/v ovalbumin per eye) followed by intravenous administration of $[^{99m}Tc]$-human serum albumin (0.25 mCi). Animals were then challenged with antigen either once or twice (2nd challenge 24 hours later). The response was a marked increase in vascular permeability as measured by extravasation of $[^{99m}Tc]$-labelled human serum albumin into the conjunctiva.

To measure the inhibitory effect of Compounds I-IV, at varying times before antigen challenge, Compounds I-IV were administered either i.v. (1 mg/kg dosage) or topically (0.1% suspensions or solutions in 10 µl of 0.5% hydroxy ethylcellulose). The results, expressed as percentage inhibition of the antigen induced inflammation response, are shown in Tables 7-1 and 7-2.

## TABLE 7-1

### INHIBITION OF ANTIGEN-INDUCED VASCULAR PERMEABILITY
### CHANGES IN GUINEA-PIG CONJUNCTIVA
### FOLLOWING SINGLE CHALLENGE

| Compound | Pre-treat. Time (min.) | % Inhib. | ± Sem* | No. of Exper. |
|---|---|---|---|---|
| I | | | | |
| (i.v.) | 2 | 1.5 | 25.9 | 7 |
| (topical) | 5 | 1.7 | 13.7 | 7 |
| II | | | | |
| (topical) | 5 | 0 | 11.9 | 7 |
| III | | | | |
| (topical) | 5 | 38.8*** | 5.8 | 8 |
| IV | | | | |
| (topical) | 5 | 28.8** | 10.0 | 8 |

\* Standard Error of the Mean

\*\* $p < 0.01$

\*\*\* $p < 0.001$

## TABLE 7-2

### INHIBITION OF ANTIGEN-INDUCED VASCULAR
### PERMEABILITY CHANGES IN
### GUINEA-PIG CONJUNCTIVA FOLLOWING SECOND CHALLENGE

| Compound | Pre-treat. Time (min.) | % Inhib. | ± Sem* | No. of Exper. |
|---|---|---|---|---|
| I | | | | |
| (i.v.) | 2 | 42.2** | 6.1 | 5 |
| (topical) | 5 | 37.0** | 6.5 | 6 |
| II | | | | |
| (topical) | 5 | 30.3* | 10.9 | 9 |
| III | | | | |
| (topical) | 5 | 48.0* | 12.7 | 7 |
| IV | | | | |
| (topical) | 60 | 59.5* | 7.8 | 5 |

\* $p < 0.05$

\*\* $p < 0.01$

WHAT IS CLAIMED IS:

1.    The use of a compound selected from the group consisting of:

I      4-((3-(4-acetyl-3-hydroxy-2-propylphenoxy)-propyl)sulfonyl)-γ-oxobenzene butanoic acid;

II     (βS*,γR*)-4-(3-(4-acetyl-3-hydroxy-2-propyl-phenoxy)propylthio)-γ-hydroxy-β-methylbenzene-butanoic acid;

III    4-bromo-2,7-dimethoxy-3H-phenothiazin-3-one; and

IV     2,3-dihydro-6-(3-(2-hydroxymethyl)phenyl-2-propenyl)-5-benzofuranol;

or the ophthalmologically acceptable salt thereof;   for the manufacture of a medicament useful for the treatment of allergic conjunctivitis.

2.    The use according to Claim 1 wherein the compound is administered in a polymeric insert.

3.    The use according to Claim 2 where said insert comprises from 0.001 to 10% by weight of the compound.

4.    The use according to Claim 1 wherein the compound is administered in an ointment base.

5.    The use according to Claim 1 wherein the compound is administered in a liquid vehicle.

6.    The use according to Claim 1 wherein the compound is administered in an aqueous gel.

7.   An ophthalmic composition for the topical treatment of allergic conjunctivitis comprising an effective amount of a compound selected from the group consisting of:

I   4-((3-(4-acetyl-3-hydroxy-2-propylphenoxy)-propyl)sulfonyl)-γ-oxobenzene butanoic acid;

II   (βS*,γR*)-4-(3-(4-acetyl-3-hydroxy-2-propyl-phenoxy)propylthio)-γ-hydroxy-β-methylbenzene-butanoic acid;

III   4-bromo-2,7-dimethoxy-3H-phenothiazin-3-one;  and

IV   2,3-dihydro-6-(3-(2-hydroxymethyl)phenyl-2'-propenyl)-5-benzofuranol;

or the ophthalmologically acceptable salt thereof.

8.   The composition of Claim 7 wherein the compound is 2,3-dihydro-6-(3-(2-hydroxymethyl)phenyl-2-propenyl)-5-benzofuranol.

9.   The composition of Claim 7 which comprises from 0.001 to 10% by weight of the compound.